# EUROPEAN PATENT SPECIFICATION

(11) **EP 1 401 442 B1**
(45) Date of publication and mention of the grant of the patent: **17.11.2004**
(21) Application number: 02776528.8
(22) Date of filing: 06.06.2002
(51) Int. Cl.: A61K 31/4439, A61K 31/724

(54) **S-OMEPRAZOLE (ESOMEPRAZOLE) INCLUSION COMPLEX WITH CYCLODEXTRINS**
S-OMEPRAZOL (ESOMEPRAZOL)-INKLUSIONSKOMPLEX MIT CYCLODEXTRINEN
COMPOSE D'INCLUSION DE S-OMEPRAZOLE (ESOMEPRAZOLE) AVEC DES CYCLODEXTRINES

(30) Priority: 06.06.2001 GB 0113792
(43) Date of publication of application: 31.03.2004
(73) Proprietor: Cipla Ltd., Mumbai 400 008 (IN)
(72) Inventor: HAMIED, Yusuf, Khwaja, Mumbai 400026 (IN); RAO, Dharmaraj, Ramachandra, Thane 400 601, Mahara shtra (IN)
(74) Representative: Wain, Christopher Paul
(86) International application number: PCT/GB2002/002542
(87) International publication number: WO 2002/098423

(56) References cited:
- EP-A- 1 018 340
- WO-A-01/14367
- WO-A-01/28558
- WO-A-86/00913
- WO-A-93/13138
- WO-A-96/02535
- WO-A-96/38175
- WO-A-98/40069
- US-A- 5 877 192
- ARIAS M J ET AL: "STUDY OF OMEPRAZOLE-GAMMA-CYCLODEXTRIN COMPLEXATION IN THE SOLID STATE" DRUG DEVELOPMENT AND INDUSTRIAL PHARMACY, NEW YORK, NY, US, vol. 3, no. 26, March 2000 (2000-03), pages 253-259, XP008005796 ISSN: 0363-9045
- DATABASE BIOSIS [Online] BIOSCIENCES INFORMATION SERVICE, PHILADELPHIA, PA, US; 1993 RHEE GYE JU; HWANG SUNG-JOO; LEE KI MYUNG: "Complexation and properties of omeprazole with hydroxypropyl-beta-cyclodextrin." Database accession no. PREV199497128504 XP002209099
- CASTILLO J A ET AL: "Preparation and characterization of albendazole beta-cyclodextrin complexes." DRUG DEVELOPMENT AND INDUSTRIAL PHARMACY. UNITED STATES DEC 1999, vol. 25, no. 12, December 1999 (1999-12), pages 1241-1248, XP001095115 ISSN: 0363-9045
- TINWALLA A Y ET AL: "Solubilization of thiazolobenzimidazole using a combination of pH adjustment and complexation with 2-hydroxypropyl-beta-cyclodextrin." PHARMACEUTICAL RESEARCH. UNITED STATES AUG 1993, vol. 10, no. 8, August 1993 (1993-08), pages 1136-1143, XP001095116 ISSN: 0724-8741

## Description

The present invention relates to an inclusion complex, particularly, but not exclusively to an inclusion complex of S-omeprazole, and to a method of making it.

The compound omeprazole (5-methoxy-2-[[(4-methoxy-3,5-dimethyl-2-pyridinyl)methyl]sulfinyl]-1H-benzimidazole) and therapeutically acceptable salts thereof are well known as effective gastric acid secretion inhibitors, and are useful as anti-ulcer agents. Omeprazole has two enantiomeric forms, the R and S- enantiomers, otherwise known as R-omeprazole and S-omeprazole, and normally exists as a racemic mixture. Certain optically pure salts of R and S omeprazole are described for example in US 5714504. The magnesium salt of S-omeprazole trihydrate is described in WO 98/54171, and S-omeprazole in a neutral, solid form (which can be in a partly or substantially crystalline state) is described in WO 98/28294. The optical isomers of omeprazole (in particular the S-enantiomer) are believed to possess certain advantages over the racemic form - for example, the optically pure salts of omeprazole disclosed in WO 94/27988 are said to have improved pharmacokinetic properties which give an improved therapeutic profile such as a lower degree of inter-individual variation. However, one particular problem with S-omeprazole, as with other similar benzimidazole compounds, is that it is not stable in its free form. Thus, for example, the compound is readily degraded by moisture and under neutral and acidic conditions. Previous approaches to providing a stable form of S-omeprazole have concentrated on the provision of alkali metal or alkaline earth metal salts of S-omeprazole (see WO 94/27988 and WO 98/54171), but these approaches are not entirely satisfactory since the salts *per se* are still liable to degradation. Another problem with S-omeprazole in its free form is that it is difficult to isolate. It can be isolated as a trihydrate having about 13 to 15% moisture content, although this form has to be stored under refrigerated conditions to provide even limited stability.

We have now found that, surprisingly, S-omeprazole and related benzimidazoles can be provided in a form which is both stable and easily isolated and processed with minimal risk of degradation.

According to the present invention, there is provided an inclusion complex comprising a substantially pure optical isomer of a benzimidazole compound such as omeprazole, lansoprazole, pantoprazole or rabeprazole, and cyclodextrin. Preferably, the optically pure isomer is the S isomer and most preferably it is S-omeprazole. The cyclodextrin is preferably β-cyclodextrin.

The term "substantially pure optical isomer" in the context of the present invention means the S isomer when substantially free of the R isomer (or vice versa), preferably with an enantiomeric excess (e.e.) of 90% and more preferably 95% e.e.

In a further aspect, the invention provides a process for preparing an inclusion complex comprising a substantially pure optical isomer of a benzimidazole compound and cyclodextrin, which process comprises adding a cyclodextrin to an aqueous solution of a substantially pure optical isomer of a benzimidazole compound or a pharmaceutically acceptable salt thereof, and isolating the inclusion complex so formed from the solution. It is preferred to keep the solution at an alkaline pH throughout the process (i.e. a pH of above 7) so as to avoid any degradation of the active compound. The process is preferably used to prepare a β-cyclodextrin complex of S-omeprazole, but it can also be applied to other substituted benzimidazoles such as S-lansoprazole, S-pantoprazole and S-rabeprazole.

The present method enables S-omeprazole and the S isomers of other benzimidazole compounds to be prepared in a stable form, which form has much greater resistance to degradation than either the S isomers in their free form or as salts. The inclusion complex of the invention can be easily isolated in the form of a stable white powder by the present process, and this powder in turn has the advantage of excellent handleability. It can, for example, be processed easily and conveniently into final dosage forms without the need to take special precautions to stabilise the active material during processing.

US 5399700 discloses a method for stabilising a racemic mixture of an acid-unstable compound such as omeprazole by forming an inclusion complex of racemic omeprazole with cyclodextrin. EP 1018340 A teaches a method of stabilising a racemic mixture of a benzimidazole compound by forming an inclusion compound comprising a racemic benzimidazole derivative with one or more amino acids and one or more cyclodextrins. However, it should be noted that both of these disclosures relate to racemic benzimidazoles and there is no teaching about either the S or R isomers. It is well known that the behaviour and properties of optically pure isomers (particularly in terms of stability) can vary markedly from that of the racemic compound. Thus, racemic omeprazole is a free powder having a high melting point, which can be purified by normal solvent crystallisation techniques, dried free of solvents and can be handled easily at room temperature and formulated into dosage forms. However, S-omeprazole is a low melting point solid which cannot be easily purified using solvent crystallisation methods since it has a tendency to hold the solvent molecule (as a solvate), thus drying the product becomes extremely difficult. S-omeprazole can be isolated from water only as a trihydrate (as noted above). Any attempt to further dry the product so that it is free of water results in decomposition of the product. The trihydrate form is stable only under refrigerated conditions, making it almost impossible for it to be formulated into dosage forms directly. There has been no previous disclosure of attempts to solve the stability problems associated with optical isomers (particularly the S-isomers) of the benzimidazoles in the manner disclosed by the present invention. Accordingly, an inclusion complex comprising the S isomer of a benzimidazole such as omeprazole and cyclodextrin is new. In particular, we have found that it is not necessary to use a benzimidazole amino acid derivative as described in EP 1018340 to obtain excellent stability of the S isomer.

In addition, whilst other known complexes of a pharmaceutically active material and cyclodextrin can be made even by physical mixing, slurrying, kneading together as a dough etc of the active substance and cyclodextrin in any proportion, these methods cannot be applied to a complex of S-omeprazole and cyclodextrin owing to the stability problem. The present invention, however, provides a suitable process for preparing a complex of cyclodextrin with an unstable optical isomer of a benzimidazole compound, such as S-omeprazole.

The invention is described hereinafter with reference to S-omeprazole, it being understood that the invention also applies to the optical isomers of other benzimidazole compounds such as S-lansoprazole, S-pantoprazole and S-rabeprazole, and also to the R isomers of all these compounds.

S-omeprazole can be prepared by procedures well known in the art, such as those described and referred to in WO 94/27988. In forming the inclusion complex, the S-omeprazole can be used either in its free form or in the form of a pharmaceutically acceptable salt, such as the potassium salt.

It is possible to use any one of the cyclodextrins to form the inclusion complex, but we prefer to use β-cyclodextrin.

The proportion of S-omeprazole and cyclodextrin used is important in order to form a stable complex. We prefer to use a molar ratio of S-omeprazole to β-cyclodextrin in the range 1:1.5 to 1:5, with a ratio of 1:2 being particularly preferred. Reducing the amount of cyclodextrin much below these levels causes unwanted discoloration of the product to arise.

The process is preferably carried out under alkaline conditions by using an aqueous alkaline solution containing, for example, sodium hydroxide, to which the S-omeprazole is added. In principle, any alkaline substance can be employed so long as it does not interfere with the formation of the inclusion complex. Alkali metal hydroxides such as sodium hydroxide are particularly suitable. The S-omeprazole can be added to the alkaline solution either in solid form (such as a powder) or in the form of an aqueous solution. Preferably, the temperature of the solution is above at least 30°C, more preferably above 40°C. A temperature of around 45°C is ideal.

In the next stage, cyclodextrin, preferably as β-cyclodextrin, is added to the alkaline solution of S-omeprazole. It is preferred to add the cyclodextrin in small quantities over a period of about one hour. The mixture is preferably stirred thoroughly throughout the addition. The mixture is then preferably further diluted by the addition of water in order to provide a clear solution. The dilution ensures that the cyclodextrin is completely dissolved and is entirely available for complex formation. Preferably, the dilution is at least 1 in 4 by volume of the initial solution. After dilution, the pH of the mixture is then checked and preferably adjusted to between 8 to 9. For example, this can be carried out using a 5% aqueous solution of boric acid, although other equivalent means can be used.

Preferably the mixture is then cooled, most preferably to around 5°C. Cooling enables maximum recovery of the product. The inclusion complex is then isolated. This can be done, for example, by filtering the complex from the cooled solution. The inclusion complex is thus isolated in the form of a white powder. The inclusion complex can be formulated into final dosage forms such as tablets, capsules and the like using standard excipients. A particularly preferred dosage formulation is that described in our publication WO 98/52564.

The following examples illustrate the invention:

### Example 1

To an aqueous solution of sodium hydroxide (5.5 g NaOH in 1 litre) maintained at about 45°C is added an aqueous solution of potassium S-omeprazole (54 g in 200 ml). To this solution is further added β-cyclodextrin (495 g) in small quantities over a period of about 1 hour. The mass is then further diluted with 3.5 litres of water to obtain an almost clear solution. The pH of the mass is then adjusted to between 8 to 9 using a 5% aqueous solution of boric acid. The contents are cooled to 5°C and filtered to obtain 420 g of an inclusion complex of S-omeprazole with β-cyclodextrin. The complex contains about 10 to 14% of the active ingredient and is in the form of a white powder.

### Example 2

To an aqueous solution of sodium hydroxide (12.5 g in 1 litre), maintained at about 45°C is added 45 g of S-omeprazole. To this solution is further added β-cyclodextrin (495 g) in small quantities over a period of about 1 hour. The mass is then further diluted with 3.5 litres of water to obtain an almost clear solution. The pH of the mass is then adjusted to between 8 to 10 using a 5% aqueous solution of boric acid. The contents are then cooled to 5°C and filtered to obtain 400 g of an inclusion complex of S-omeprazole with β-cyclodextrin. The complex contains about 8 to 11% of the active ingredient and is in the form of a white powder.

## Claims

1. A stable inclusion complex comprising substantially pure S-omeprazole and a cyclodextrin.

2. A stable inclusion complex comprising substantially pure S-omeprazole and a cyclodextrin, wherein the complex is prepared by adding a cyclodextrin to substantially pure S-omeprazole or a pharmaceutically acceptable salt thereof, and precipitating the inclusion complex so formed from the solution.

3. A complex according to claim 1 or 2, wherein the cyclodextrin is ∃-cyclodextrin.

4. A pharmaceutical composition comprising an inclusion complex according to any of claims 1 to 3, and a pharmaceutically acceptable carrier therefor.

5. A process for preparing a stable inclusion complex comprising substantially pure S-omeprazole and a cyclodextrin, which process comprises adding a cyclodextrin to substantially pure S-omeprazole, or a pharmaceutically acceptable salt thereof, and precipitating the inclusion complex so formed from the solution.

6. A process according to claim 5, wherein the aqueous solution is an aqueous alkaline solution.

7. A process according to claim 5 or 6, wherein before isolation of the inclusion complex, the process further comprises the steps of diluting the mixture containing the said complex and adjusting the pH.

8. A process according to claim 7, wherein the pH is adjusted to between 8 and 10 using an aqueous solution of boric acid.

9. A process according to any of claims 5 to 8, wherein the temperature of the solution/mixture is maintained at 45°C or above.

10. A process according to any of claims 5 to 9, wherein before isolation of the inclusion complex the mixture is cooled to 5°C or below.

11. A process for preparing an inclusion complex according to any of claims 1 to 3 substantially as described in Example 1 or Example 2.

12. Use of an inclusion complex according to any of claims 1 to 3 for the manufacture of a medicament for treating gastric acid-related diseases and gastro intestinal inflammatory diseases in animals and man, such as gastric ulcer, duodenal ulcer, reflux oesophagitis and gastritis.

## Patentansprüche

1. Beständige Einschlussverbindung umfassend im Wesentlichen reines S-Omeprazol und ein Cyclodextrin.

2. Beständige Einschlussverbindung umfassend im Wesentlichen reines S-Omeprazol und ein Cyclodextrin, wobei die Verbindung durch Zusetzen eines Cyclodextrins zu im Wesentlichen reinem S-Omeprazol oder einem pharmazeutisch akzeptablen Salz desselben und Ausfällen der so gebildeten Einschlussverbindung aus der Lösung zubereitet wird.

3. Verbindung nach Anspruch 1 oder 2, wobei es sich beidem Cyclodextrin um β-Cyclodextrin handelt.

4. Pharmazeutische Zusammensetzung umfassend eine Einschlussverbindung nach einem der Ansprüche 1 bis 3 und einen pharmazeutisch akzeptablen Träger dafür.

5. Verfahren für das Zubereiten einer beständigen Einschlussverbindung umfassend im Wesentlichen reines S-Omeprazol und ein Cyclodextrin, welches Verfahren das Zusetzen eines Cyclodextrins zu im Wesentlichen reinem S-Omeprazol oder einem pharmazeutisch akzeptablen Salz desselben und das Ausfällen der so gebildeten Einschlussverbindung aus der Lösung umfasst.

6. Verfahren nach Anspruch 5, wobei die wässrige Lösung eine wässrige alkalische Lösung ist.

7. Verfahren nach Anspruch 5 oder 6, wobei das Verfahren vor dem Isolieren der Einschlussverbindung des Weiteren die Schritte des Verdünnens der die Verbindung enthaltenden Mischung und das Einstellend des pH-Werts umfasst.

8. Verfahren nach Anspruch 7, wobei der pH-Wert mit Hilfe einer wässrigen Lösung von Borsäure auf 8 bis 10 eingestellt wird.

9. Verfahren nach einem der Ansprüche 5 bis 8, wobei die Temperatur der Lösung/Mischung bei 45 °C oder darüber gehalten wird.

10. Verfahren nach einem der Ansprüche 5 bis 9, wobei die Mischung vor dem Isolieren des Einschlussprodukts auf 5 °C oder weniger gekühlt wird.

11. Verfahren für das Zubereiten einer Einschlussverbindung nach einem der Ansprüche 1 bis 3 im Wesentlichen wie in Beispiel 1 oder Beispiel 2 beschrieben.

12. Verwendung einer Einschlussverbindung nach einem der Ansprüche 1 bis 3 für die Herstellung eines Medikaments für die Behandlung mit Magensäure verbundener Krankheiten und entzündlicher Magen-Darmerkrankungen bei Tieren und dem Menschen wie beispielsweise Magengeschwür, Zwölffingerdarmgeschwür, Refluxösophagitis und Magenschleimhautentzündung.

## Revendications

1. Complexe d'inclusion stable comprenant du S-oméprazole sensiblement pur et une cyclodextrine.

2. Complexe d'inclusion stable comprenant du S-oméprazole sensiblement pur et une cyclodextrine, dans lequel on prépare le complexe en ajoutant une cyclodextrine dans du S-oméprazole sensiblement pur ou un sel pharmacologiquement acceptable de celui-ci, et en précipitant le complexe d'inclusion ainsi formé à partir de la solution.

3. Complexe selon la revendication 1 ou 2, dans lequel la cyclodextrine est une β-cyclodextrine.

4. Composition pharmaceutique comprenant un complexe d'inclusion selon l'une quelconque des revendications 1 à 3, et un porteur pharmacologiquement acceptable pour celui-ci.

5. Procédé de préparation d'un complexe d'inclusion stable comprenant du S-oméprazole sensiblement pur et une cyclodextrine, lequel procédé consiste à ajouter une cyclodextrine à du S-oméprazole sensiblement pur, ou un sel pharmacologiquement acceptable de celui-ci, et à précipiter le complexe d'inclusion ainsi formé à partir de la solution.

6. Procédé selon la revendication 5, dans lequel la solution aqueuse est une solution alcaline aqueuse.

7. Procédé selon la revendication 5 ou 6, dans lequel avant isolation du complexe d'inclusion, le procédé comprend en outre les phases de dilution du mélange contenant ledit complexe et de réglage de l'acidité pH.

8. Procédé selon la revendication 7, dans lequel on ajuste l'acidité pH entre 8 et 10 à l'aide d'une solution aqueuse d'acide borique.

9. Procédé selon l'une quelconque des revendications 5 à 8, dans lequel la température de la solution/du mélange est maintenue supérieure ou égale à 45°C.

10. Procédé selon l'une quelconque des revendications 5 à 9, dans lequel avant isolation du complexe d'inclusion, on refroidit le mélange à une température inférieure ou égale à 5°C.

11. Procédé de préparation d'un complexe d'inclusion selon l'une quelconque des revendications 1 à 3 sensiblement selon la description de l'exemple 1 ou de l'exemple 2.

12. Utilisation d'un complexe d'inclusion selon l'une quelconque des revendications 1 à 3 pour la fabrication d'un médicament destiné à traiter les maladies liées à l'acide gastrique et les maladies inflammatoires du système gastro-intestinal chez les animaux et chez l'homme, comme l'ulcère gastrique, l'ulcère du duodénum, l'oesophagite à reflux et la gastrite.
